Europäisches Patentamt

European Patent Office

Office européen des brevets

(19)

(11) **EP 0 942 718 B1**

(12) **FASCICULE DE BREVET EUROPEEN**

(45) Date de publication et mention
de la délivrance du brevet:
**27.07.2005 Bulletin 2005/30**

(21) Numéro de dépôt: **97933713.6**

(22) Date de dépôt: **11.07.1997**

(51) Int Cl.7: **A61K 31/24**, A61K 9/28

(86) Numéro de dépôt international:
**PCT/FR1997/001281**

(87) Numéro de publication internationale:
**WO 1998/005320 (12.02.1998 Gazette 1998/06)**

(54) **COMPRIME DE MALEATE DE TRIMEBUTINE PELLICULE**

UMHÜLLTE TABLETTE ENTHALTEND TRIMEBUTIN MALEAT

COATED TRIMEBUTINE MALEATE TABLET

(84) Etats contractants désignés:
**AT CH ES FR GR IT LI PT**
Etats d'extension désignés:
**RO**

(30) Priorité: **07.08.1996 FR 9609956**

(43) Date de publication de la demande:
**22.09.1999 Bulletin 1999/38**

(73) Titulaire: **Pfizer Holding France**
**75014 Paris (FR)**

(72) Inventeurs:
• **BENKERROUR, Loutfy**
**F-75015 Paris (FR)**
• **DOAT, Bernard, Résidence la Pléiade**
**F-49000 Angers (FR)**

(74) Mandataire: **Dekker, Henrike et al**
**Pfizer**
**European Pharma Patent Department**
**23-25, avenue du Docteur Lannelongue**
**75668 Paris Cedex 14 (FR)**

(56) Documents cités:
**EP-A- 0 076 428        EP-A- 0 076 515**
**EP-A- 0 099 109        FR-A- 2 468 364**
**FR-A- 2 506 613        FR-A- 2 640 876**

• **DATABASE WPI Week 9204 Derwent**
**Publications Ltd., London, GB; AN 92-028855**
**[04] XP002030108 cité dans la demande & JP 03**
**275 622 A (TEISAN TEIYAKU KK) 6 décembre**
**1991**
• **DATABASE WPI Week 9616 Derwent**
**Publications Ltd., London, GB; AN 96-157001**
**XP002030109 cité dans la demande & JP 08 040**
**885 A (TANABE SEIYAKU CO.) 13 février 1996**

**Description**

Domaine de l'invention

La présente invention concerne un comprimé de maléate de trimébutine amélioré et pelliculé destiné à l'administration par voie orale et son procédé de préparation.

En particulier, l'invention concerne un nouveau comprimé qui permet d'administrer une quantité thérapeutiquement efficace de maléate de trimébutine pour une mise à disposition biologique rapide ou prolongée, mais, en tout état de cause, sans provoquer d'effet amer persistant qui, à terme, nuit à la bonne observance du traitement par le patient.

Arrière-plan technologique de l'invention

La trimébutine, ou 3,4,5-triméthoxybenzoate de 2-diméthylamino-2-phényl-*n*-butyle, est le premier médicament connu pour agir sur les récepteurs enképhalinergiques périphériques, notamment digestifs. C'est un régulateur de la motricité digestive. Telle qu'elle, ou salifiée par l'acide maléique, la trimébutine est proposée sous forme de comprimé, de solution injectable, de suppositoire et de poudre pour préparation d'une suspension buvable. Internationalement commercialisées, ces diverses formes pharmaceutiques sont indiquées dans le traitement des douleurs liées aux troubles fonctionnels du tube digestif et des voies biliaires, et dans le traitement des douleurs et de l'inconfort liés aux troubles fonctionnels intestinaux.

Les formes administrables par voie orale sont plus particulièrement adaptées aux traitements ambulatoires. Ce sont essentiellement les comprimés et aussi les granulés ou les poudres qui peuvent être destinés à la préparation de suspensions buvables.

Le maléate de trimébutine est le principe actif couramment utilisé pour la préparation de ces formes orales avec une posologie indicative journalière de 300 à 600 mg pour l'adulte. Ce produit, obtenu par cristallisation dans l'eau, fond à 105-106°C. Sa solubilité dans l'eau à 25°C est environ de 1 % (p/v) alors que la trimébutine base y est insoluble.

La trimébutine est, du point de vue chimique, un ester de type benzoate sensible aux agents 30 favorisant les réactions d'hydrolyse ou celles apparentées. Ces agents comprennent notamment l'eau et les solvants réactifs comme les alcools inférieurs, la température, la lumière et divers catalyseurs en particulier métalliques qu'ils soient alcalins ou alcalino terreux. L'hydrolyse de la trimébutine conduit, entre autres sous produits, à la formation d'acide 3,4,5-triméthoxybenzoïque dont la détermination quantitative permet d'apprécier sa stabilité chimique dans diverses conditions de conservation.

Egalement l'acide maléique est thermosensible de par sa propension à s'isomériser en acide fumarique dont la constante d'acidité est inférieure à celle de l'acide maléique. Cet acide, par salification avec la trimébutine conduit à un fumarate de trimébutine de faible solubilité dans l'eau.

Par ailleurs, comme nombre de composés chargés positivement en solution aqueuse à des pH proches de la neutralité, le maléate de trimébutine, administré par voie orale, induit dans la cavité et sur les muqueuses buccales une forte amertume et une sensation astringente durable qui peut, à terme, provoquer une mauvaise observance des traitements dans lesquels le produit est prescrit. Cette amertume est particulièrement pénalisante lorsqu'il est nécessaire d'obtenir un effet thérapeutique rapide ce qui suppose une mise à disposition biologique quasi immédiate dès que le médicament a été dégluti. Pour illustration de la réalité de ce problème, une des deux spécialités sous forme comprimée commercialisée en France indique que "les comprimés doivent être avalés sans être croqués, avec un verre d'eau" (dictionnaire des spécialités pharmaceutiques "Vidal" - ed. 1996)

A ce jour, l'état de la technique ne rapporte que des tentatives imparfaites pour surmonter les problèmes exposés, à savoir à la fois assurer la stabilité du maléate de trimébutine lors de la préparation et la conservation des médicaments et masquer ou éliminer, au niveau buccal, l'amertume persistante provoquée par le produit sans en retarder l'effet thérapeutique.

En ce qui concerne la forme poudre le brevet FR 2 468 364 publié le 8/5/1981 décrit un procédé de préparation de microcapsules contenant un composé pharmaceutiquement actif dont les parois sont l'éthylcellulose, procédé réalisé dans le cyclohexane et qui fait intervenir un agent inducteur de séparation de phases, préférentiellement de la classe des phospholipides. Pour exemple il est préparé des microcapsules de maléate de trimébutine pour lesquelles un des critères de qualité évalué est l'absence de goût amer.

Selon ce même procédé réalisé dans le cyclohexane les brevets qui suivent visent à perfectionner la qualité des microcapsules:

- FR 2 506 613 publié le 3/12/1982 décrit des microcapsules dont les parois sont constituées d'éthylcellulose et d'un polymère insoluble dans l'eau mais soluble en milieu acide qui visent une libération rapide du produit en milieu

EP 0 942 718 B1

gastrique . La quantité de maléate de trimébutine incorporée n'excède pas 15 %, la libération de 50 % du produit en milieu gastrique artificiel étant obtenue après 17 minutes.

- EP 0 076 515 publié le 13/4/1983 concerne aussi des microcapsules à libération rapide en milieu gastrique dont les parois sont constituées d'éthylcellulose et d'un polymère soluble dans l'eau et/ou en milieu acide. La quantité de produit incluse est au mieux de 12 % dont 50 % sont libérés en 10 à 20 minutes.
- EP 0 076 428 publié également le 13/4/1983 vise le même but que les brevets précédents, les parois sont constituées d'éthylcellulose et d'un polymère "gonflable" dans l'eau. Elles contiennent environ 10 % de principe actif dont 50 % sont libérés en 4 à 25 minutes en milieu gastrique artificiel.
- EP 0 099 109 publié le 25/1/1984 décrit un procédé annoncé avantageux de préparation de microcapsules dans lequel intervient un agent inducteur de séparation de phase particulier.

Il est clair qu'à travers cette succession de brevets leur demanderesse vise la préparation d'une poudre contenant à l'état enrobé du maléate de trimébutine afin d'en masquer l'amertume tout en tentant d'obtenir une libération rapide du principe actif à partir des microcapsules proposées. Il apparaît que ce but soit imparfaitement atteint puisque d'une part ce procédé, complexe et économiquement insatisfaisant, met en oeuvre le cyclohexane qui est un solvant inflammable, difficile à éliminer et soumis au titre de solvant résiduel à des normes rigoureuses par les autorités de santé, et que, d'autre part il conduit à des microcapsules qui ne contiennent qu'une quantité de maléate de trimébutine inférieure à 20%, ce qui nécessite aux patients d'absorber une quantité excessivement importante de médicament, compte tenu de la posologie journalière préconisée de 300 à 600 mg de maléate de trimébutine, puisque contenant une quantité plus de quatre fois supérieure d'excipients que de principe actif.

A la demande JP 80 40 885 publiée le 13/2/1996 il est décrit une formulation composée d'un mélange de granulés dans laquelle, pour ceux contenant le maléate de trimébutine, le goût amer est pratiquement éliminé par granulation du principe actif avec un polymère insoluble dans l'eau et un sel hydrophobe. Pour exemple, des granulés comprenant le maléate de trimébutine contiennent 20 % du principe actif, 3 % de polymère insoluble et 5 % d'un sel hydrophobe à savoir le stéarate de magnésium ou le stéarate de calcium. Il n'est mentionné aucun résultat concernant la vitesse de libération et/ou la stabilité du maléate de trimébutine dans ces granulés.

En ce qui concerne les comprimés destinés à l'administration orale du maléate de trimébutine, au brevet FR 2 640 876 publié le 29/06/1990, il est décrit une composition pharmaceutique sous forme d'un comprimé exempt d'enrobage et comprenant, comme principe actif du maléate de triméthoxy-3,4,5 benzoate de diméthylamino-2-phényl-2-butanol-I, caractérisée en ce que ce principe actif, qui représente de 35 à 45 % en poids de la composition est dispersé d'une manière homogène dans une matrice poreuse hydrophile d'hydroxypropylméthylcellulose représentant de 15 à 20 % en poids de la composition qui comprend en outre de 20 à 25 % en poids d'un diluant hydrosoluble et de 10 à 20 % en poids d'acide tartrique.

Selon cette invention la cinétique de dissolution du principe actif est, entre trente minutes et 8 heures apparentée à l'ordre zéro, 50 % environ du produit étant libéré après 8 heures.

Il est à remarquer que ces comprimés, notamment ceux fortement dosés, peuvent être difficilement déglutis par certains patients, et que, dans ces conditions, la durée de contact involontairement prolongée dans la cavité buccale provoque inévitablement une amertume désagréable et persistante.

La demande JP N° 73574/1990 publiée le 6/12/1991 vise une préparation solide très semblable à celle du brevet FR 2 640 876 et des comprimés de maléate de trimébutine destinée à l'administration orale, dans lesquels un acide organique, à raison de 0,1 à 20 mg pour 100 mg de maléate, inhibe significativement la décomposition du produit, décomposition dans laquelle le stéarate de magnésium est partiellement impliqué. Il est précisé que les acides convenables sont l'acide tartrique ou l'acide citrique et que la formulation comprend un stéarate métallique (magnésium ou calcium) comme agent lubrifiant pour la compression.

Description de l'invention

Surmontant les problèmes irrésolus tels qu'exposés dans l'état de la technique, la présente invention est une solution techniquement simple, économiquement avantageuse et bénéfique au patient qui a pour objet un nouveau comprimé amélioré de maléate de trimébutine destiné à l'administration par voie orale, caractérisé en ce qu'il comprend :

un noyau comprenant, en poids, de 30 à 60 % de maléate de trimébutine, et, à titre d'excipients, de 15 à 45 % d'un ou plusieurs agents diluants, de 2,5 à 25 % d'un ou plusieurs agents liants, de 0,5 à 25 % d'un ou plusieurs agents acidifiants choisi parmi l'acide citrique et l'acide tartrique, optionnellement de 1 à 5 % d'un ou plusieurs agents désagrégants, et des agents d'utilité technique à savoir de 0,5 à 5 % d'un agent d'écoulement et de 0,25 à 2,5% d'un agent lubrifiant, et un pelliculage hydrosoluble représentant de 1 à 5 % en poids de la matrice, la composition dudit pelliculage comprenant un ou plusieurs agents filmogènes hydrosolubles, un ou plusieurs agents opacifiants, et, optionnellement, un ou plusieurs agents plastifiants.

Tel que décrit dans ce mémoire, dans son aspect principal, l'invention concerne un nouveau comprimé pour l'administration par voie orale d'une quantité thérapeutiquement efficace de maléate de trimébutine dont la mise à disposition biologique peut être rapide ou prolongée mais, en tout état de cause, ne provoquant dans la cavité buccale aucune amertume persistante, qui à l'usage provoque une attitude répulsive du patient. Ces comprimés présentent une résistance et une stabilité physique remarquable qui les rend aptes aux conditionnements les plus divers (blisters, vrac en flacons...) dans des conditions de conservation éprouvantes (température, humidité...)

Egalement un aspect important concerne la remarquable stabilité chimique du maléate de trimébutine présenté sous cette forme de comprimés, même conservés dans des conditions rigoureuses, ce qui assure aux patients une sécurité et un bénéfice thérapeutique certain.

Par un autre aspect, l'invention vise le procédé de fabrication des comprimés.

Un autre aspect de l'invention concerne une composition appropriée à la préparation des noyaux des comprimés caractérisée en ce que l'adaptation quantitative et qualitative de ses constituants principaux permet d'obtenir une libération rapide ou prolongée du principe actif.

De façon générale le pelliculage des comprimés procure des avantages tant pour leur fabrication industrielle que pour leur utilisation par le patient. Ainsi, en ce qui concerne l'aspect fabrication, les formes pelliculées sont reconnues être moins sensibles aux érosions mécaniques, donc générer moins de poussières et, de ce fait, diminuer dans les locaux les contaminations inter-médicaments ; également il est reconnu que le pelliculage favorise la mise en conditionnement primaire, notamment sous blisters, des comprimés. En ce qui concerne l'utilisation par les patients, la texture satinée ou lustrée apportée par le mélange filmogène du pelliculage évite l'adhérence du comprimé nu à la muqueuse buccale et rend la déglutition plus aisée.

Outre ces avantages connus, les améliorations apportées par les comprimés de l'invention sont développées dans la suite de ce mémoire et illustrées par des essais de libération du principe actif et de stabilités, physique et chimique, comparés avec deux spécialités commercialisées.

Au sens le plus large de l'invention, les comprimés sont caractérisés en ce que leurs noyaux comprennent, en poids de 30 à 60% de maléate de trimébutine, et, à titre d'excipients, de 15 à 45% d'un ou plusieurs agents diluants, de 2,5 à 25% d'un ou plusieurs agents liants, de 0.5 à 25% d'un ou plusieurs agents acidifiants, optionnellement de 1 à 5% d'un ou plusieurs agents désagrégants, et des agents d'utilité technique à savoir de 0,5 à 5% d'un agent d'écoulement et de 0,25 à 2,5% d'un agent lubrifiant et caractérisée en ce que lesdits noyaux sont enrobés d'un film représentant de 1 à 5% en poids dudit comprimé, film dont la composition comprend un ou plusieurs agents filmogènes hydrosolubles, un ou plusieurs agents opacifiants, et, optionnellement, un ou plusieurs agents plastifiants.

Plus précisément la composition d'un noyau comprend :

- à raison de 20 à 40%, un ou plusieurs agents diluants choisis parmi la cellulose sous forme microcristalline ou de poudre, le mannitol, l'amidon, et plus particulièrement, le lactose, notamment hydraté, qui est l'agent de dilution préféré.

- à raison de 2,5 à 25%, un ou plusieurs agents liants choisi parmi la cellulose microcristalline, la gélatine de qualité pharmaceutiquement acceptable, la méthylcellulose, l'amidon prégélatinisé. La povidone, l'amidon de mais prégélatinisé et les hydroxypropyl méthylcellulose de viscosité comprise entre 2,5 et 15.000 mPa.s sont préférés. Particulièrement, pour les noyaux dont on souhaite une désagrégation rapide on utilise un mélange représentant de 5 à 10% du comprimé et composé de 5 à 10 parties d'amidon de mais et de 0,5 à 2 parties d'hydroxypropylméthyl cellulose dont la viscosité est comprise entre 2,5 et 17,5 mPa.s alors que pour les noyaux pour lesquels on souhaite une libération prolongée du principe actif on utilise à raison de 15 à 20% du comprimé un mélange composé de 5 à 15 parties d'hydroxypropylméthyl cellulose dont la viscosité est comprise entre 1.500 et 15.000 mPa.s et de 0,5 à 1 partie de povidone. De façon particulièrement préférée on utilise pour les noyaux à désagrégation rapide l'hydroxypropylméthyl cellulose de viscosité de 5,2 à 7,0 mPa.s et, pour les noyaux à libération prolongée l'hydroxypropylméthylcellulose de viscosité 4.000 mPa.s.

- l'agent acidifiant est choisi parmi les acides organiques pharmaceutiquement acceptables, connus pour leurs propriétés séquestrantes des métaux et/ou possédant un effet stabilisant sur le principe actif et/ou permettant de créer un environnement acide favorable à la libération du principe actif des noyaux hydrophiles visant une libération prolongée. Ces acides sont l'acide citrique et l'acide tartrique, qui, dans les noyaux dont on souhaite une désagrégation rapide, sont utilisés à raison de 0,5 à 2%, et, dans les noyaux pour lesquels on souhaite une libération prolongée du maléate de trimébutine, sont utilisés de préférence à raison de 10 à 20% notamment avec l'acide tartrique qui est préféré.

- l'agent d'écoulement est le dioxyde de silice, anhydre ou hydraté, que l'on utilise à raison de 0,75 à 2,5%.

- l'agent lubrifiant est choisi parmi les sels métalliques de l'acide stéarique à l'exclusion des autres agents habituellement proposés pour cet effet. L'agent lubrifiant est ainsi choisi parmi les stéarates de calcium ou de magnésium. Ce dernier est préféré à des concentrations de 0,5 à 1,5% et plus particulièrement de 1 %,

- l'agent désagrégant, qui est optionnel, est choisi parmi la crospovidone (polyvidone réticulée), le sel de sodium de la carboxyméthyl cellulose réticulée, également dénommée croscarmellose sodique" et le carboxyméthylamidon qui est préféré. Le caractère optionnel de cet agent correspond à l'éventuelle nécessité de libérer rapidement le principe actif ce qui est obtenu par la désagrégation rapide des noyaux après passage de la sphère bucco-pharyngée. A cet effet pour les comprimés dits "à libération immédiate" on utilise la carboxyméthylamidon sodique à raison de 1 à 5% et de préférence 2% pour obtenir la désagrégation escomptée.

Et, en ce qui concerne le film d'enrobage, celui-ci est essentiellement constitué d'un agent filmogène hydrosoluble choisi parmi les polymères acryliques ou les polymères cellulosiques comme l'hydroxypropylméthyl cellulose de viscosité comprise entre 2,5 et 17,5 mPa.s, la méthylcellulose, l'hydroxypropylcellulose ; d'une substance de charge permettant une meilleure adhérence du film au noyau comme le lactose ou la cellulose microcristalline. Les agents opacifiants et/ou colorants additifs sont choisis parmi les oxydes de fer ou le dioxyde de titane. Les agents plastifiants utilisables sont choisis parmi les macrogols poids moléculaire moyen de 3000 à 6000, le propylène glycol, la glycérine ou le polyoxyl 40 stéarate. Pour la mise en oeuvre du pelliculage des noyaux, selon l'invention on peut utiliser les composés mélangés en solution ou en suspension dans l'eau ou dans des solvants pharmaceutiquement acceptables tels que l'éthanol, l'acétone ou leurs mélanges avec l'eau. On préfère toutefois un mélange comprenant une suspension aqueuse d'hydroxypropylméthyl cellulose dont la viscosité est comprise entre 2.5 et 17,5 mPa.s et de dioxyde de titane ou encore un mélange comprenant une hydroxypropylméthyl cellulose dont la viscosité est comprise entre 2,5 et 17,5 mPa.s, du lactose ou de la cellulose microcristalline, du polyéthylène glycol de poids moléculaire 4000 ou du polyoxyl 40 stéarate et du dioxyde de titane en suspension aqueuse. On préfère particulièrement les mélanges dans lesquels l'hydroxypropylméthyl cellulose soit de viscosité de 5,2 à 7,0 mPa.s; de tels mélanges, particulièrement appropriés, sont commercialisés par la société Colorcon sous la marque OPADRY® ou par la société Seppic sous la marque SEPIFILM®, ils permettent d'obtenir à raison de 1,5 à 3% en poids par rapport au poids du noyau et particulièrement à raison de 2% un film de résistance transitoire en milieu bucco-pharyngé qui permet d'administrer le comprimé sans effet d'amertume tout en préservant une mise à disposition biologique du principe actif dans les conditions de libération choisies pour celui-ci. Par ailleurs le pelliculage réalisé par le film dans les conditions de l'invention respecte parfaitement les gravures, en creux ou en relief, des noyaux, gravures indicatives du principe actif et de son dosage et qui peuvent, au cours de l'opération d'enrobage avec des compositions inappropriées être soit comblées, soit érodées ou érasées et dans tous les cas devenir illisibles.

A titre particulièrement préféré l'invention vise les comprimés dont les compositions centésimales sont les suivantes :

Comprimés A de désagrégation rapide dits formes à libération immédiate : "FLI"

i) composition pour noyaux de 200 & 400 mg

- maléate de trimébutine        50,00%
- lactose monohydraté        36,00%
- amidon de mais        7,00%
- hydroxypropylméthyl cellulose (6 mPa.s)        1,00%
- carboxyméthyl amidon sodique        2,00%
- acide tartrique        1,00%
- gel de silice        2,00%
- stéarate de magnésium        1,00%

ii) enrobage 2,00% du poids du noyau d'une composition comprenant hydroxypropylméthyl cellulose (6 mPa.s), lactose, polyéthylène glycol 4000 et dioxyde de titane, ou de façon alternative hydroxypropylméthyl cellulose (6 mPa.s), cellulose microcristalline, polyoxyl 40 stéarate et dioxyde de titane.

Comprimés B dits à libération prolongée : "FLP"

i) composition pour noyaux de 747,00 & 508,00 mg

- maléate de trimébutine        39,37%
- lactose monohydraté        23,6 %

- hydroxypropylméthyl cellulose (4000mPa.s)     15,75 %
- acide tartrique     15,75%
- povidone     1,57%
- gel de silice     1,00%
- stéarate de magnésium 1,00%

ii) <u>enrobage</u> 2,00% du poids du noyau d'une composition comprenant hydroxypropylméthyl cellulose (6 mPa.s) et dioxyde de titane ou de façon alternative hydroxypropylméthyl cellulose (6 mPa.s), lactose, polyéthylène glycol 4000 et dioxyde de titane.

En ce qui concerne le procédé de fabrication des comprimés selon l'invention, celui-ci consiste dans un premier temps à préparer une composition appropriée, puis à la soumettre à la compression pour obtenir les noyaux qui sont ensuite pelliculés.

Ainsi, la mise en oeuvre du procédé de préparation des comprimés dits "FLI" dont les constituants préférés ont été décrits précédemment pour les comprimés A consiste :

i) à la préparation de la composition

- dans un mélangeur à poudre ou un appareil à lit fluidisé mélanger le maléate de trimébutine, le lactose monohydraté, l'amidon de maïs prégélatinisé, l'hydroxypropylméthyl cellulose et la moitié du carboxyméthylamidon, puis granuler le mélange homogène avec l'acide tartrique en solution aqueuse et calibrer les granulés humides,
- sécher en lit fluidisé ou en étuve ventilée à 60°C les granulés, puis les calibrer sur tamis de 1 à 1,5 mm d'ouverture, puis les mélanger dans un mélangeur à poudre avec la seconde moitié du carboxyméthylamidon, le gel de silice et le stéarate de magnésium.

ii) à préparer les noyaux par compression des granulés sur pastilleuse rotative avec des poinçons de taille appropriée pour obtenir des noyaux de masse équivalente à 200 ou 400 mg, dosées respectivement à 100 et 200 mg de maléate de trimébutine et dont la dureté est de 50 à 70 N,

iii) à pelliculer les noyaux en turbine avec la suspension de pelliculage à une température comprise entre 35 et 45°C et dans des conditions telles que ce pelliculage recouvre uniformément les noyaux à raison d'une quantité de 4 mg pour les noyaux de 200 mg et 8 mg pour les noyaux de 400 mg.

La mise en oeuvre du procédé de préparation des comprimés B dits "FLP", peu différente de celle précédemment exposée, consiste :

i) à la préparation de la composition

- dans un mélangeur à poudre ou un appareil à lit fluidisé mélanger le maléate de trimébutine, le lactose monohydraté, et l'acide tartrique puis granuler le mélange homogène avec une solution aqueuse ou éthanolique de povidone et calibrer les granulés humides,
- sécher en lit fluidisé ou en étuve ventilée à une température de 45 à 60°C les granulés, les calibrer sur tamis de 1 à 1,5 mm d'ouverture, puis les mélanger dans un mélangeur à poudre avec l'hydroxypropylméthyl cellulose, le gel de silice et le stéarate de magnésium.

ii) à préparer les noyaux par compression des granulés sur pastilleuse rotative avec des poinçons de taille appropriée pour obtenir des noyaux de masse équivalente à 747 ou 508 mg, dosées respectivement à 300 et 200 mg de maléate de trimébutine et dont la dureté est de 80 à 200 N,

iii) à pelliculer les noyaux en turbine avec la suspension de pelliculage à une température comprise entre 35 et 45°C et dans des conditions telles que ce pelliculage recouvre uniformément les noyaux à raison d'une quantité de 15 mg pour les noyaux de 747 mg et 10 mg pour les noyaux de 508 mg.

Tels que préparés les comprimés pelliculés sont ensuite conditionnés sous blisters, en flacons polypropylène ou polyéthylène haute densité, etc...

L'invention est illustrée de façon non limitative par les exemples qui suivent.

Exemple 1 : comprimés pelliculés de désagrégation rapide dosés à 100 mg de maléate de trimébutine - forme dite "FLI"

Dans un mélangeur de poudre à projection et tourbillonnement on introduit :

- 10,400 kg de maléate de trimébutine,
- 7,488 kg de lactose monohydraté,
- 1,450 kg d'amidon de mais prégélatinisé,
- 0,208 kg de carboxyméthylamidon sodique,
- 0,208 kg d'hydroxypropylméthyl cellulose (6 mPa.s).

Le mélange homogène des constituants est réalisé par agitation durant 3 minutes, puis, dans le même appareil en utilisant l'outil adapté à la granulation, on mouille le mélange par une solution de 0,208 kg d'acide tartrique en solution dans 2,7 l d'eau.
Les granulés sont calibrés humides sur calibreur rotatif puis séchés sur lit fluidisé à 50'C jusqu'à ce que l'humidité résiduelle soit inférieure à 1%.
Les granulés sont alors calibrés sur tamis rapide d'ouverture de maille 1,5 mm puis introduits dans un mélangeur de poudre, type container. On ajoute alors :

- 0,208 kg de carboxyméthylamidon,
- 0,416 kg de gel de silice,
- 0,208 kg de stéarate de magnésium.

L'ensemble est mélangé durant 20 minutes à vitesse lente (5 à 10 tours/minute) puis soumis à la compression sur presse à comprimer rotative équipée de poinçons de diamètre 8 mm. Les noyaux obtenus ont les caractéristiques suivantes :

- masse moyenne        200 mg $\pm$ 15 mg
- dureté moyenne        60 N $\pm$ 10 N
- friabilité        < 1 %
- temps de désagrégation dans l'eau à 37°C : maximum 8 minutes.

Les noyaux sont ensuite pelliculés en turbine (par exemple dans un appareil de type "ultra coater aeromatic $S_2$") par pulvérisation d'une suspension aqueuse contenant de la méthylhydroxypropyl cellulose (6 mPa.s), du lactose hydraté, du dioxyde de titane et du polyéthylène glycol 4000. Dans le type d'appareil précité les conditions de l'opération sont les suivantes :

- température d'entrée d'air : 50°C,
- débit d'introduction de la suspension de pelliculage : 30 g/minute,
- pression de pulvérisation : 2 bars,

pour obtenir à une température de 35 à 45°C, un pelliculage homogène de 4 mg par comprimé dont la masse finale moyenne est de 204 mg $\pm$ 15,3 mg et dont la composition unitaire est la suivante :

- maléate de trimébutine        100,0 mg
- lactose monohydraté        72,0 mg
- amidon de mais prégélatinisé        14,0 mg
- hydroxypropylméthyl cellulose        2,0 mg
- carboxyméthylamidon sodique        4,0 mg
- acide tartrique        2,0 mg
- gel de silice        4,0 mg
- stéarate de magnésium        2,0 mg
- mélange d'agents de pelliculage        4,0 mg

dont hydroxypropylméthyl cellulose 6 mPa.s 0,93 mg, lactose hydraté 1,4 mg, dioxyde de titane 0,87 mg et polyéthylène glycol 4000 0,80 mg.
Le conditionnement primaire de ces comprimés est effectué en plaquettes thermoformées (aluminium-chlorure de polyvinyle).
Un mode opératoire identique est utilisé pour préparer les comprimés "FLI" dosé à 200 mg de maléate de trimébutine. La modification apportée consiste à équiper la presse à compression rotative de poinçons appropriés pour obtenir des noyaux d'une masse moyenne de 400 mg, ce qui, dans les conditions de l'exemple est réalisé avec des poinçons dont le diamètre est de 11 mm. Ces noyaux sont ensuite pelliculés par le mélange d'agents de pelliculage, à raison de 8 mg par noyau afin d'obtenir des comprimés finis d'une masse moyenne de 408 mg.

Exemple 2 : comprimés pelliculés à libération prolongée dosés à 300 mg de maléate de trimébutine - forme dite "FLP"

Dans un appareil à lit fluidisé type "Aéromatic" on introduit :

- 10,500 kg de maléate de trimébutine,
- 2,100 kg de lactose monohydraté
- 4,200 kg d'acide tartrique,

après homogénéisation on ajoute progessivement 2,000 kg d'une solution aqueuse à 21 % (p/v) de povidone et on poursuit le mélange durant 16 minutes. Le grain obtenu est séché à 60°C jusqu'à ce que la solvatation résiduelle, déterminée à la thermobalance, soit inférieure à 0,6 %.
Le granulé est alors calibré sur tamis rapide d'ouvenure de maille 1,5 mm puis introduit dans un mélangeur de poudre à projection et tourbillonnement. On introduit alors 4,200 kg de lactose atomisat, 4,200 kg d'hydroxypropylméthyl cellulose 4000 mPa.s, 262,5 g de gel de silice et 262,5 g de stéarate de magnésium.
L'ensemble est alors mélangé durant 2 minutes puis soumis à la compression sur presse à comprimer rotative. Les noyaux obtenus ont les caractéristiques suivantes :

- masse moyenne       747 mg ± 30 mg
- dureté moyenne      180 N ± 20 N
- friabilité       < 1 %

Les noyaux sont ensuite pelliculés en turbine comme décrit à l'exemple 1 avec une suspension aqueuse contenant l'hydroxypropylméthyl cellulose (6 mPa.s), et du dioxyde de titane pour obtenir un pelliculage homogène de 15 mg par comprimé dont la masse finale moyenne est de 762 mg ± 30 mg et dont la composition est la suivante :

- maléate de trimébutine       300,0 mg
- lactose monohydraté       180,0 mg
- acide tartrique       120,0 mg
- polyvidone       12,0 mg
- hydroxypropylméthyl cellulose       120,0 mg
- gel de silice       7,5 mg
- stéarate de magnésium       7,5 mg
- mélange d'agents de pelliculage 15,0 mg

dont hydroxypropylméthylcellulose 6 mPa.s 11,5 mg et dioxyde de titane 3,5 mg.
Les comprimés sont conditionnés en plaquettes thermoformées ou en flacons selon leur utilisation et les conditions de stockage.
Une variante de ce mode opératoire consiste à équiper la presse à comprimer rotative de poinçons adaptés pour obtenir des noyaux dont la masse moyenne est de 508,0 mg dosées à 200,0 mg de maléate de trimébutine. Ces noyaux sont pelliculés avec le mélange d'agents de pelliculage à raison de 10,0 mg par noyaux pour obtenir des comprimés finis dont la masse moyenne est de 518,0 mg.

Essais

Les essais réalisés comprennent :

- l'étude de la stabilité physique et chimique des comprimés pelliculés à libération rapide ou prolongée de l'invention,
- l'étude comparative des comprimés pellicules à libération rapide de l'invention avec des comprimés commercialisés.

Excepté l'appréciation de l'aspect, les essais concernant les critères physiques ont été réalisés selon des protocoles adaptés de la Pharmacopée européenne qui sont les essais :

- de désagrégation (Ph. Eur. V.5.1.1),
- de dissolution (Ph. Eur. V.5.4.1),
- de friabilité (Ph. Eur. V.5.8.2.-1),
- de dureté (Ph. Eur. V.5.8.3.-1).

En ce qui concerne les études de stabilité chimique du maléate de trimébutine, celles-ci ont été déterminées par le dosage de l'acide 3,4,5-triméthoxybenzoïque qui est représentatif de l'hydrolyse du principe actif. Ce dosage est réalisé par chromatographie liquide haute pression (CLHP - Ph. Eur. V.6.20.4) avec une colonne de 125 mm d'un support type Lichrospher 60 RP sélect B Merck et en pratiquant l'élution avec un mélange tampon phosphate pH 3,6 - acétonitrile, la détection spectrophotométrique étant effectuée à 220 nm.

- études de stabilité

Sous des conditionnements en plaquettes thermoformées (PVC - aluminium) ou en flacons de polypropylène les comprimés pelliculés "FLI" et "FLP" étudiés ont été conservés 6 mois en étuves à 40°C ± 4°C, en atmosphère anhydre ou d'humidité relative (HR) de 75% ± 5%. Les résultats de ces essais sont rapportés aux tableaux qui suivent.

1) Stabilité des comprimés FLI

Les études ont été réalisées après 6 mois de conservation des comprimés à 40 °C ± 4 °C en atmosphère d'humidité relative de 75 % ± 5 %.
Les critères retenus pour l'étude sont :

- l'aspect, le temps de désagrégation, la dureté, l'effritement et l'essai de dissolution du principe actif en ce qui concerne la stabilité physique,
- le dosage de l'ATMB en ce qui concerne la stabilité chimique du principe actif.

| | To | T 6 mois 40 °C + 75 % HR |
|---|---|---|
| **Stab. physique** | | |
| - aspect | blanc | blanc cassé |
| - dureté | 79 N | 82 N |
| - effritement | 1,27 % | 0,69 % |
| - temps de désagrégation | 7 à 9 mn | 10 à 12 mn |
| - essai dissolution 10 mn | 25 % | 20,7 % |
| - essai dissolution 20 mn | 100,3 % | 96,9 % |
| - essai dissolution 30 mn | 100,9 % | 101,8 % |
| **Stab. chimique** | | |
| - % ATMB | 0,01 % | 0,10 % |

2) Stabilité des comprimés FLP

Les études ont été réalisées après 6 mois de conservation des comprimés à 40 °C ± 4 °C en milieu anhydre et en atmosphère d'humidité relative de 75 % ± 5 %.
Les critères retenus pour l'étude sont :

- l'aspect et l'essai de dissolution pour la stabilité physique,
- le dosage de l'ATMB pour la stabilité chimique du principe actif.

| | To | T 6 mois | |
|---|---|---|---|
| | | 40 °C | 40 °C + 75 % HR |
| **Stab. physique** | | | |
| - aspect | blanc | blanc cassé | blanc cassé |
| - dissolution   1 h | 7,6 % | 7,3 % | |
| 4 h | 25,8 % | 27,7 % | |
| 8 h | 49,0 % | 54,8 % | |
| **Stab. chimique** | | | |
| - % ATMB | 0,09 % | 0,10 % | 0,15 % |

Ces essais, effectués dans des conditions de conservation rigoureuses, sont probants de l'excellente stabilité des comprimés de l'invention.
En ce qui concerne les comprimés "FLI" :

- la stabilité physique s'avère remarquable notamment en considérant le critère significatif du but visé par l'invention qui est d'éviter la désagrégation prématurée du comprimé et, en conséquence, la dissolution du principe actif. En effet, on observe sur ces points après 6 mois une légère augmentation du temps de désagrégation (environ 3 minutes) qui correspond à un léger recul de la vitesse de dissolution du maléate de trimébutine,
- la stabilité chimique est également considérée satisfaisante.

La formation de 0,09 % d'ATMB correspond aux rapports des poids moléculaires à la dégradation de $\frac{0,09 \times 503,5}{212,2} = 0,21$ % de maléate de trimébutine.
(ATMB - PM = 212,2 ; maléate de trimébutine - PM = 503,5)
En ce qui concerne les comprimés "FLP" :

- la stabilité physique est remarquable en considérant le critère de dissolution qui est essentiel pour les formes à libération prolongée,
- la stabilité chimique est également bonne en considérant que la formation de 0,06 % d'ATMB correspond à la dégradation de 0,14 % de maléate de trimébutine.

- essais comparatifs

Pour preuve des améliorations notables apportées par la forme pelliculée suivant l'invention il a été réalisé des essais comparatifs entre les comprimés obtenus à l'exemple 1 et des comprimés commercialisés dosés à 100,0 mg de maléate de trimébutine.
En ce qui concerne ces derniers, l'information qualitative des excipients des deux spécialités figure au dictionnaire des spécialités pharmaceutiques "Vidal" :

- comprimés "D" : lactose, mannitol, saccharose, polyéthylèneglycol 6000, stéarate de magnésium, gélatine, amidon de blé, gel de silice.
- comprimés "T" : lactose, hypromellose, croscarmellose, stéarate de magnésium.

Les comprimés de l'exemple 1 ont été comparés avec les comprimés "D" et/ou les comprimés "T" en ce qui concerne d'une part, la libération du principe actif et d'autre part, leur stabilité chimique après conservation à 40°C - 75% d'hygrométrie durant 6 mois en ce qui concerne l'hydrolyse du maléate de trimébutine déterminée par dosage d'acide 3,4,5 triméthoxy benzonique (ATMB).

Les résultats sont présentés dans les tableaux qui suivent :

|  | Ex. 1 | Cpmé "D" | Cpmé "T" |
|---|---|---|---|
| **% dissolution** |  |  |  |
| - 10 mn | 23,0 % | 26,0 % | 83,3 % |
| - 20 mn | 101,2 % | 50,4 % | 98,7 % |
| - 30 mn | 101,7 % | 69,2 % | 98,6 % |
| - 45 mn | - | 92,9 % |  |
| - 60 mn | - | 98,0 % |  |

**Etude comparée de dissolution**

| | Etudes à 6 mois  40 °C - 75 % HR | | |
|---|---|---|---|
| | Ex. 1 | Cpmé "D" | Cpmé "T" |
| - % ATMB | 0,10 % | 0,45 % | 0,70 % |
| - % dégrad. principe actif (*) | 0,23 % | 1,06 % | 1,66 % |

**Etude comparée de stabilité**

(*) % relatif de dégradation calculé au rapport des PM de l'ATMB (212,2) et du maléate de trimébutine (503,5) et en faisant abstraction de l'ATMB présent à To.

Ces essais sont probants des améliorations apportées par le comprimé pelliculé selon l'invention :

- l'étude de dissolution prouve l'efficacité transitoire du pelliculage qui permet de libérer la quasi totalité du principe actif entre 10 et 20 minutes alors que pour les comprimes "D" la totalité de la libération n'est obtenue qu'après 60 minutes et pour les comprimés "T" cette libération est pratiquement totale avant 10 minutes. Ces résultats montrent que les comprimés "D", pour éviter l'amertume buccale, ne libèrent le principe actif que lentement et de façon excessivement retardée alors que, contrairement, les comprimés "T" libèrent le maléate de trimébutine de façon excessivement rapide avec pour conséquence l'appartition de l'amertume buccale persistante.
- l'étude de stabilité chimique montre que, globalement, la dégradation du maléate de trimébutine est respectivement 4 et 6 fois plus importante dans les comprimés "D" et "T" que dans les comprimés de l'invention suivant l'exemple 1.

**Revendications**

1. Comprimé pelliculé de maléate de trimébutine destiné à l'administration par voie orale, **caractérisé en ce qu'**il comprend un noyau comprenant, en poids, de 30 à 60 % de maléate de trimébutine, et, à titre d'excipients, de 15 à 45 % d'un ou plusieurs agents diluants, de 2,5 à 25 % d'un ou plusieurs agents liants, de 0,5 à 25 % d'un ou plusieurs agents acidifiants choisi parmi l'acide citrique et l'acide tartrique, optionnellement de 1 à 5 % d'un ou plusieurs agents désagrégants, et des agents d'utilité technique à savoir de 0,5 à 5 % d'un agent d'écoulement et de 0,25 à 2,5% d'un agent lubrifiant, et un pelliculage hydrosoluble représentant de 1 à 5 % en poids de la matrice, la composition dudit pelliculage comprenant un ou plusieurs agents filmogènes hydrosolubles, un ou plusieurs agents opacifiants, et, optionnellement, un ou plusieurs agents plastifiants.

2. Comprimé pelliculé selon la revendication 1 **caractérisé en ce que** l'agent acidifiant est l'acide tartrique à raison de 0,5 à 2 %, dans les noyaux pour libération rapide du maléate de trimébutine et à raison de 10 à 20 % dans les noyaux pour libération prolongée.

3. Comprimé pelliculé selon l'une des revendications 1 à 2 **caractérisé en ce que** le pelliculage est choisi entre un mélange d'hydroxypropylméthyl cellulose de viscosité comprise entre 2,5 et 17,5 mPa.s et de dioxyde de titane et un mélange d'hydroxypropylméthyl cellulose de viscosité comprise entre 2,5 et 17,5 mPa.s, de lactose ou de cellulose microcristalline, de polyéthylène glycol de poids moléculaire 4000 ou de polyoxyl 40 stéarate et de dioxyde de titane.

4. Comprimé pelliculé de désagrégation rapide dont la composition centésimale pour des noyaux de 200 et 400 mg est :

    i) noyaux

    - maléate de trimébutine        50,00%
    - lactose monohydraté        36,00%
    - amidon de maïs        7,00%
    - hydroxypropylméthyl cellulose (6 mPa.s)        1,00%
    - carboxyméthyl amidon sodique        2,00%
    - acide tartrique        1,00%
    - gel de silice        2,00%
    - stéarate de magnésium        1,00%

    ii) enrobage 2,00% du poids du noyau d'une composition comprenant hydroxypropylméthyl cellulose (6 mPa.s), lactose, polyéthylène glycol 4000 et dioxyde de titane, ou de façon alternative hydroxypropylméthyl cellulose (6 mPa.s), cellulose microcristalline, polyoxyl 40 stéarate et dioxyde de titane.

5. Comprimé pelliculé à libération prolongée dont la composition centésimale pour des noyaux de 747,00 et 508,00 mg est :

    i) noyaux

    - maléate de trimébutine        39,37%
    - lactose monohydraté        23,6%
    - hydroxypropylméthyl cellulose (4000mPa.s)        15,75%
    - acide tartrique        15,75%
    - povidone        1,57%
    - gel de silice        1,00%
    - stéarate de magnésium        1,00%

    ii) enrobage 2,00% du poids du noyau d'une composition comprenant hydroxypropylméthyl cellulose (6 mPa.s) et dioxyde de titane ou de façon alternative hydroxypropylméthyl cellulose (6 mPa.s), lactose, polyéthylène glycol 4000 et dioxyde de titane.

6. Procédé de préparation de comprimés pelliculés selon la revendication 1 qui consiste :

i) à préparer le mélange des composants des noyaux

- par mélange du maléate de trimébutine et des excipients dans un mélangeur à poudre ou un appareil à lit fluidisé puis par granulation du mélange homogène avec l'agent acidifiant en solution aqueuse et calibrer les granulés humides,
- sécher en lit fluidisé ou en étuve ventilée à 60°C les granulés, puis les calibrer et les mélanger dans un mélangeur à poudre avec le gel de silice et le stéarate de magnésium.

ii) à préparer les noyaux par compression des granulés sur pastilleuse rotative avec des poinçons de taille appropriée,
iii) à pelliculer les noyaux en turbine avec la suspension de pelliculage à une température comprise entre 35 et 45°C et dans des conditions telles que ce pelliculage recouvre uniformément les noyaux à raison d'une quantité de 2% de leur poids.

**Patentansprüche**

1. Trimebutinmaleat-Filmtablette zur Verabreichung auf oralem Weg, **dadurch gekennzeichnet, dass** sie einen Kern umfassend, in Gewichtsprozent, 30 bis 60 % Trimebutinmaleat und als Exzipienten 15 bis 45 % eines oder mehrerer Verdünnungsmittel, 2,5 bis 25 % eines oder mehrerer Bindemittel, 0,5 bis 25 % eines oder mehrerer Säuerungsmittel ausgewählt aus Citronensäure und Weinsäure, gegebenenfalls 1 bis 5 % eines oder mehrerer zerfallsfördernder Mittel und Mittel mit technischem Nutzen, nämlich 0,5 bis 5 % eines Fließmittels und 0,25 bis 2,5 % eines Gleitmittels, und eine wasserlösliche Filmschicht aufweist, die 1 bis 5 Gew.% der Matrix ausmacht, wobei die Zusammensetzung der Filmschicht einen oder mehrere wasserlösliche Filmbildner, ein oder mehrere Trübungsmittel und gegebenenfalls ein oder mehrere Plastifizierungsmittel umfasst.

2. Filmtablette nach Anspruch 1, **dadurch gekennzeichnet, dass** das Säuerungsmittel Weinsäure in einer Menge von 0,5 bis 2 % in den Kernen für rasche Freisetzung von Trimebutinmaleat und in einer Menge von 10 bis 20 % in den Kernen für verzögerte Freisetzung ist.

3. Filmtablette nach einem der Ansprüche 1 bis 2, **dadurch gekennzeichnet, dass** die Filmschicht ausgewählt ist aus einer Mischung von Hydroxypropylmethylcellulose mit einer Viskosität zwischen 2,5 und 17,5 mPa.s und Titandioxid und einer Mischung von Hydroxypropylmethylcellulose mit einer Viskosität zwischen 2,5 und 17,5 mPa.s, Lactose oder mikrokristalliner Cellulose, Polyethylenglycol mit einem Molekulargewicht von 4000 oder Polyoxyl-40-stearat und Titandioxid.

4. Rasch lösliche Filmtablette, deren zentesimale Zusammensetzung für Kerne von 200 und 400 mg wie folgt ist:

   i) Kern

   - Trimebutinmaleat        50,00 %
   - Lactosemonohydrat        36,00 %
   - Maisstärke        7,00 %
   - Hydroxypropylmethylcellulose (6 mPa.s)        1,00 %
   - Natrium-Carboxymethylstärke        2,00 %
   - Weinsäure        1,00 %
   - Silikagel        2,00 %
   - Magnesiumstearat        1,00 %

   ii) Umhüllung: 2,00 Gew.% des Kerns mit einer Zusammensetzung umfassend Hydroxypropylmethylcellulose (6 mPa.s), Lactose, Polyethylenglycol 4000 und Titandioxid oder als Alternative Hydroxypropylmethylcellulose (6 mPa.s), mikrokristalline Cellulose, Polyoxyl-40-stearat und Titandioxid.

5. Filmtablette mit verzögerter Freisetzung, deren zentesimale Zusammensetzung für Kerne von 747,00 und 508,00 mg wie folgt ist:

   i) Kern

- Trimebutinmaleat        39,37 %
- Lactosemonohydrat        23,60 %
- Hydroxypropylmethylcellulose (4000 mPa.s)        15,75 %
- Weinsäure        15,75 %
- Povidon        1,57 %
- Silikagel        1,00 %
- Magnesiumstearat        1,00 %

ii) Umhüllung: 2,00 Gew.% des Kerns mit einer Zusammensetzung umfassend Hydroxypropylmethylcellulose (6 mPa.s) und Titandioxid oder als Alternative Hydroxypropylmethylcellulose (6 mPa.s), Lactose, Polyethylenglycol 4000 und Titandioxid.

6. Verfahren zur Herstellung von Filmtabletten nach Anspruch 1, welches darin besteht, dass

i) die Mischung der Bestandteile der Kerne hergestellt wird

- durch Mischen des Trimebutinmaleats mit den Exzipienten in einem Pulvermischer oder einer Wirbelschichtvorrichtung, anschließend Granulieren der homogenen Mischung mit dem Säuerungsmittel in wässeriger Lösung und Kalibrieren des feuchten Granulats,
- Trocknen des Grantrlats in einer Wirbelschicht oder einem ventilierten Trockenschrank bei 60°C, anschließend Malibrieren und Mischen desselben in einem Pulvermischer mit dem Silikagel und dem Magnesiumstearat;

ii) die Kerne durch Komprimieren des Granulats auf einer rundlaufenden Tablettiermaschine mit Prägestempel geeigneter Größe hergestellt werden;
iii) die Kerne in einer Turbine mit der Suspension der Filmschicht bei einer Temperatur zwischen 35 und 45°C und unter derartigen Bedingungen überzogen werden, dass die Filmschicht die Kerne in einem Mengenverhältnis von 2 % ihres Gewichts gleichmäßig umhüllt.

**Claims**

1. Film-coated trimebutine maleate tablet intended for oral administration, **characterized in that** it comprises a core comprising, by weight, 30 to 60% of trimebutine maleate, and, as excipients, 15 to 45% of one or more diluents, 2.5 to 25% of one or more binding agents, 0.5 to 25% of one or more acidifying agents chosen among citric acid and tartaric acid, optionally 1 to 5% of one or more disintegrating agents, and technical adjuvant agents, namely 0.5 to 5% of a glidant and 0.25 to 2.5% of a lubricant, and a water-soluble film coating representing 1 to 5% by weight of the matrix, the composition of said coating comprising one or more water-soluble film-forming agents, one or more opacifying agents and, optionally, one or more plasticizers.

2. Film-coated tablet according to Claim 1, **characterized in that** the acidifying agent is tartaric acid, in the proportion of 0.5 to 2% in the cores for rapid release of trimebutine maleate and in the proportion of 10 to 20% in the cores for sustained release.

3. Film-coated tablet according to any of Claims 1 and 2, **characterized in that** the film coating is chosen from a mixture of hydroxypropylmethylcellulose of viscosity between 2.5 and 17.5 mPa.s and titanium dioxide, and a mixture of hydroxypropylmethylcellulose of viscosity between 2.5 and 17.5 mPa.s, lactose or microcrystalline cellulose, polyethylene glycol of molecular weight 4000 or polyoxyl 40 stearate and titanium dioxide.

4. Film-coated tablet for rapid disintegration, whose percentage composition for cores weighing 200 and 400 mg is:

i) <u>cores</u>

- trimebutine maleate        50.00%
- lactose monohydrate        36.00%
- maize starch        7.00%
- hydroxypropylmethylcellulose (6 mPa.s)        1.00%
- sodium carboxymethyl starch        2.00%

- tartaric acid        1.00%
- silica gel       2.00%
- magnesium stearate        1.00%

ii) <u>coating</u> 2.00% of the weight of the core of a composition comprising hydroxypropylmethylcellulose (6 mPa. s), lactose, polyethylene glycol 4000 and titanium dioxide, or alternatively hydroxypropylmethylcellulose (6 mPa.s), microcrystalline cellulose, polyoxyl 40 stearate and titanium dioxide.

5. Film-coated sustained-release tablet whose percentage composition for cores weighing 747.00 and 508.00 mg is:

i) <u>cores</u>

- trimebuline maleate        39.37%
- lactose monohydrate        23.6%
- hydroxypropylmethylcellulose (4000 mPa.s)        15.75%
- tartaric acid        15.75%
- povidone        1.57%
- silica gel        1.00%
- magnesium stearate        1.00%

ii) <u>coating</u> 2.00% of the weight of the core of a composition comprising hydroxypropylmethylcellose (6 mPa. s) and titanium dioxide, or alternatively hydroxypropylmethylcellulose (6 mPa.s), lactose, polyethylene glycol 4000 and titanium dioxide.

6. Process for preparing the film-coated tablets according to claim 1, which consists in:

i) preparing the mixture of the components of the cores

- by mixing the trimebutine maleate and the excipients in a powder mixer or a fluidized bed apparatus, followed by granulation of the homogeneous mixture with the acidifying agent in aqueous solution and sizing of the wet granules,
- drying the granules in a fluidized bed or in a ventilated oven at 60°C, then sizing them and mixing them in a powder mixer with the silica gel and the magnesium stearate,

ii) preparing the cores by compression of the granules on a rotary pelleting machine with punches of suitable size,
iii) film-coating the cores in a coating pan with the film-coating suspension at a temperature of between 35 and 45°C and under conditions such that this film coating coats the cores uniformly in the proportion of an amount of 2% of their weight.